# EUROPEAN PATENT SPECIFICATION

(11) **EP 4 057 960 B1**
(45) Date of publication and mention of the grant of the patent: **01.01.2025**
(21) Application number: 20808523.3
(22) Date of filing: 11.11.2020
(51) Int. Cl.: A61F 13/00, A61F 13/02

(54) **WOUND SEALING FILM**
WUNDVERSCHLUSSFILM
FILM D'ÉTANCHÉITÉ DE PLAIE

(30) Priority: 11.11.2019 US 201962933688 P
(43) Date of publication of application: 21.09.2022
(73) Proprietor: KCI Manufacturing Unlimited Company, Dublin, D01C4E0 (IE)
(72) Inventor: SEDDON, James, San Antonio, Texas 78265 (US); ROBINSON, Timothy Mark, San Antonio, Texas 78265 (US); RICE, Justin, San Antonio, Texas 78265 (US); INGRAM, Shannon C., San Antonio, Texas 78265 (US); LOCKE, Christopher Brian, San Antonio, Texas 78265 (US)
(74) Representative: Simmons & Simmons
(86) International application number: PCT/IB2020/060619
(87) International publication number: WO 2021/094946

(56) References cited:
- EP-A1- 3 137 029
- WO-A1-2015/130471
- WO-A1-2019/161321
- US-A- 3 457 919
- US-A1- 2006 173 087
- US-A1- 2018 353 339

## Description

### TECHNICAL FIELD

Aspects of the present disclosure relate generally to a compound film. In particular, the invention concerns a composite film containing a first polymer layer, second adhesive layer and a third gel layer, system and method for forming the compound film and method of using the compound film for a dressing of a wound therapy system. In some aspects, the second adhesive layer and the third gel layer can be combined to form a combined gel adhesive layer.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times. Efficient sealing between the wound covering and a patient skin is desirable for negative pressure therapy.
WO2015/130471 discloses a negative pressure treatment system including a hybrid drape having a film layer, a layer of bonding adhesive, and a mesh.
US2018/353339 discloses a negative pressure dressing having a drape, a manifold, and a wound contact layer with fenestrations.

### SUMMARY

The invention is defined by the independent claim. A selection of optional features of the invention is set out in the dependent claims.

Insofar as the term invention or embodiment is used in the following, or features are presented as being optional, this should be interpreted in such a way that the only protection sought is that of the invention claimed (with due regard to Article 69 EPC and the protocol thereto). References to "embodiment(s)" throughout the description which are not under the scope of the appended claims merely represent possible exemplary executions and are not part of the present invention.

### BRIEF DESCRIPTION OF THE DRAWINGS

A further understanding of the nature and advantages of the present disclosure may be realized by reference to the following drawings. The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may nonidentical reference numbers.
**FIG. 1** is a sectional view (A) and a perspective view (B) of a wound sealing film according to an example of the current invention.
**FIG. 2** is a sectional view (A) and a perspective view (B) of a wound sealing film according to an example of the current invention. (C) and (D) top view of the third layer of according two examples showing the plurality of perforation, the residual layer is shown in black and the perforation are shown in white.
**FIG. 3** is a sectional view (A) and a perspective view (B) of a wound sealing film according to an example of the current invention.
**FIG. 4** is a sectional view (A) and a perspective view (B) of a wound sealing film according to an example of the current invention. (C) and (D) top view of the third layer of according two examples showing the plurality of perforation, the residual layer is shown in black and the perforation are shown in white.
**FIG. 5 (A)** is the wound sealing film of FIG. 1 with a first cover layer and second cover layer. (B) is the wound sealing film of FIG. 3 with a first cover layer and second cover layer.
**FIG. 6 (A)** and (B) is a diagram of an example of a therapy system including a wound sealing film.

### DETAILED DESCRIPTION

As used herein, the terms "tissue site" and "target tissue" as used herein can broadly refer to a wound (e.g., open or closed), a tissue disorder, and/or the like located on or within tissue, such as, for example, bone tissue, adipose tissue, muscle tissue, neural tissue, dermal tissue, vascular tissue, connective tissue, cartilage, tendons, ligaments, and/or the like. The terms "tissue site" and "target tissue" as used herein can also refer to a surrounding tissue area(s) and/or areas of tissue that are not necessarily wounded or exhibit a disorder, but include tissue that would benefit from tissue generation and/or tissue that may be harvested and transplanted to another tissue location. The terms "tissue site" and "target tissue" may also include incisions, such as a surgical incision. In some implementations, "target tissue" may correspond or refer to a wound, and "tissue site" may correspond or refer to a tissue area(s) surrounding and including the target tissue. Additionally, the term "wound" as used herein can refer to a chronic, subacute, acute, traumatic, and/or dehisced incision, laceration, puncture, avulsion, and/or the like, a partial-thickness and/or full thickness burn, an ulcer (e.g., diabetic, pressure, venous, and/or the like), flap, and/or graft. A wound may include chronic, acute, traumatic, subacute, and dehisced wounds, partial-thickness burns, ulcers (such as diabetic, pressure, or venous insufficiency ulcers), flaps, grafts, and fistulas, for example.

The term "positive-pressure" (or "hyperbaric") as used herein generally refers to a pressure greater than a local ambient pressure, such as the ambient pressure in a local environment external to a sealed therapeutic environment (e.g., an internal volume). In most cases, this positive-pressure will be greater than the atmospheric pressure at which the patient is located. Alternatively, the positive-pressure may be greater than a hydrostatic pressure associated with tissue at the tissue site. Unless otherwise indicated, values of pressure stated herein are gauge pressures. References to increases in positive-pressure typically refer to an increase in absolute pressure, and decreases in positive-pressure typically refer to a decrease in absolute pressure. Additionally, the process of increasing pressure may be described illustratively herein as "applying", "delivering," "distributing," "generating", or "providing" positive-pressure, for example.

The term "reduced-pressure" (and "negative-pressure" or "hypobaric") as used herein generally refers to a pressure less than a local ambient pressure, such as the ambient pressure in a local environment external to a sealed therapeutic environment (e.g., an internal volume). In most cases, this reduced-pressure will be less than the atmospheric pressure at which the patient is located. Alternatively, the reduced-pressure may be less than a hydrostatic pressure associated with tissue at the tissue site. Unless otherwise indicated, values of pressure stated herein are gauge pressures. References to increases in reduced-pressure typically refer to a decrease in absolute pressure, and decreases in reduced-pressure typically refer to an increase in absolute pressure. Additionally, the process of reducing pressure may be described illustratively herein as "applying", "delivering," "distributing," "generating", or "providing" reduced-pressure, for example.

The term "fluid" may refer to liquid, gas, air, or a combination thereof. The term "fluid seal," or "seal," means a seal adequate to maintain a pressure differential (e.g., positive-pressure or reduced-pressure) at a desired site given the particular pressure source or subsystem involved. Similarly, it may be convenient to describe certain features in terms of fluid "inlet" or "outlet" in such a frame of reference. However, the fluid path may also be reversed in some applications, such as by substituting a reduced-pressure source (negative or hypobaric pressure source) for a positive-pressure source, and this descriptive convention should not be construed as a limiting convention.

The wound sealing film disclosed herein can provide many benefits, including lower leak rates, higher moisture vapor transmission rates, highly conformability, flow-able sealing that can seal creases and leaks, high bond strength, color changing to indicate when the wound sealing film has been exposed to electromagnetic energy that increases or decreases the tackiness of the wound sealing film, high moisture vapor transfer rate over the entire surface of the film, and a lower cost.

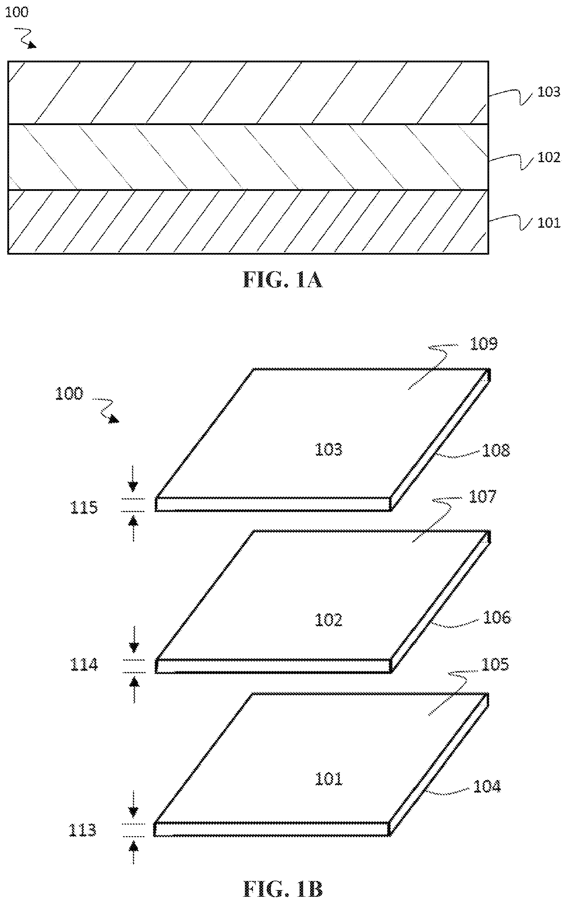

FIG. 1A illustrates a sectional view of a wound sealing film according to an example of the current invention. The wound sealing film 100, can include a first layer 101, a second layer 102 and third layer 103. The first layer 101 can be any of the first layers or liquid barrier layers described herein. The second layer 102 can be any of the second layers or adhesive layers described herein. The third layer 103 can be any of the third layers or liquid retaining polymeric film layers described herein. The second layer 102 can be positioned between the first layer 101 and the third layer 103. FIG. 1B illustrates a perspective view of the wound sealing film 100. The first layer 101 can have a first surface 104 and second surface 105. The second layer 102 can have a first surface 106 of the second surface 107. The third layer 103 can have a first surface 108 and second surface 109. The second surface 105 of first layer 101 can be in contact with the first surface 106 of the second layer 102. The second surface 107 of second layer 102 can be in contact with the first surface 108 third layer 103. In FIG. 1B the layers are shown separated for representation purpose.

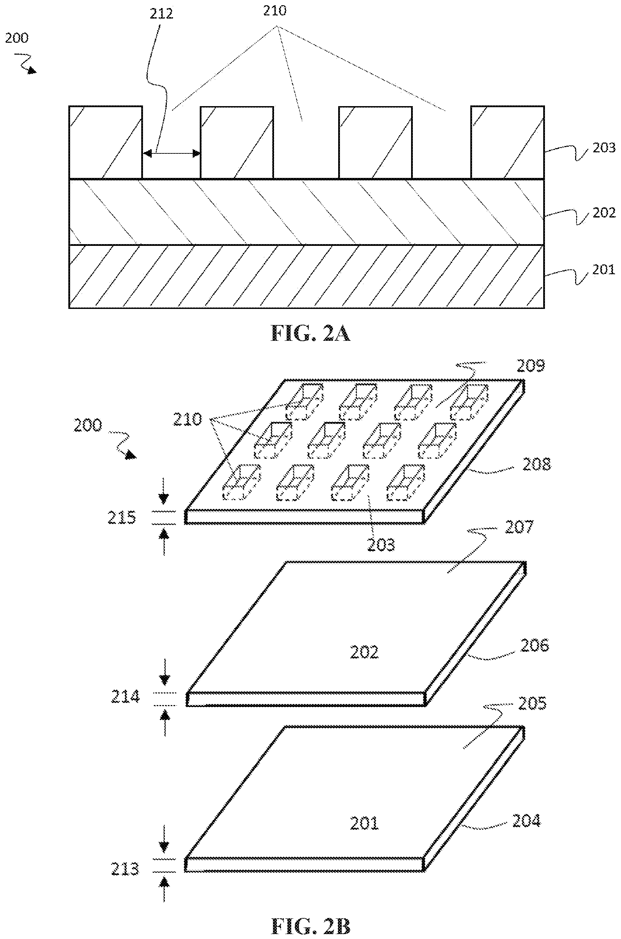

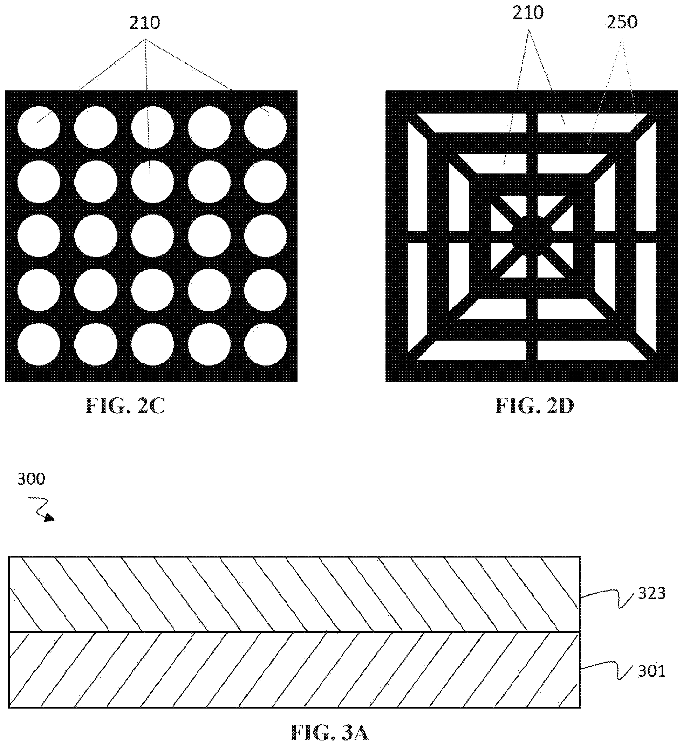

FIG. 2A illustrates a sectional view of a wound sealing film according to an example of the current invention. The wound sealing film 200, can include a first layer 201, a second layer 202 and third layer 203. The first layer 201 can be any of the first layers or liquid barrier layers described herein. The second layer 202 can be any of the second layers or adhesive layers described herein. The third layer 203 can be any of the third layers or liquid retaining polymeric film layers described herein. The second layer 202 can be positioned between the first layer 201 and the third layer 203. FIG. 2B illustrates a perspective view of the wound sealing film 200. The first layer 201 can have a first surface 204 and second surface 205. The second layer 202 can have a first surface 206 and second surface 207. The third layer 203 can have a first surface 208 and second surface 209. The second surface 205 of first layer 201 can be in contact with the first surface 206 of the second layer 202. The second surface 207 of second layer 202 can be in contact with the first surface 208 of the third layer 203. The third layer 203 can have a plurality of perforations 210. Perforations 210 of the third layer 203 can be of difference shape. FIG. 2A and B illustrate a top view of the third layer according to two examples of the current invention, the residual third layer is shown in black and the perforations of the third layer is shown in white.

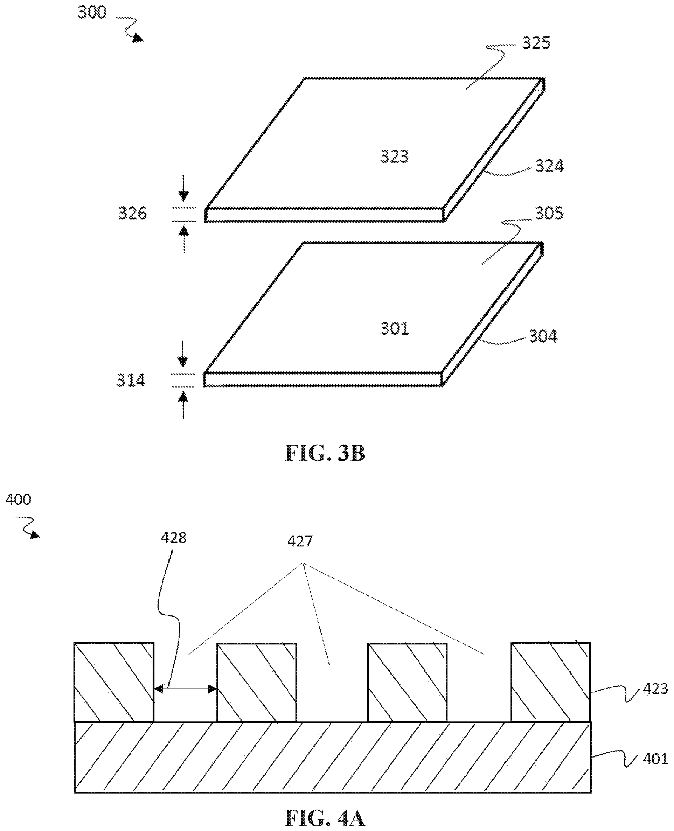

FIG. 3A illustrates a sectional view of wound sealing film according to an example of the current invention. The wound sealing film 300, can include a first layer 301, and a combined layer 323. The first layer 301 can be any of the first layers or liquid barrier layers described herein. The combined layer 323 can be any of the combined layers or polymeric gel adhesive layers described herein. FIG. 3B illustrates a perspective view of the wound sealing film 300. The first layer 301 can have a first surface 304 and second surface 305. The combined layer 323 can have a first surface 324 and second surface 325. The second surface 305 of first layer 301 can be in contact with the first surface 324 of the combined layer 323. In FIG. 3B the layers are shown separated for representation purpose.

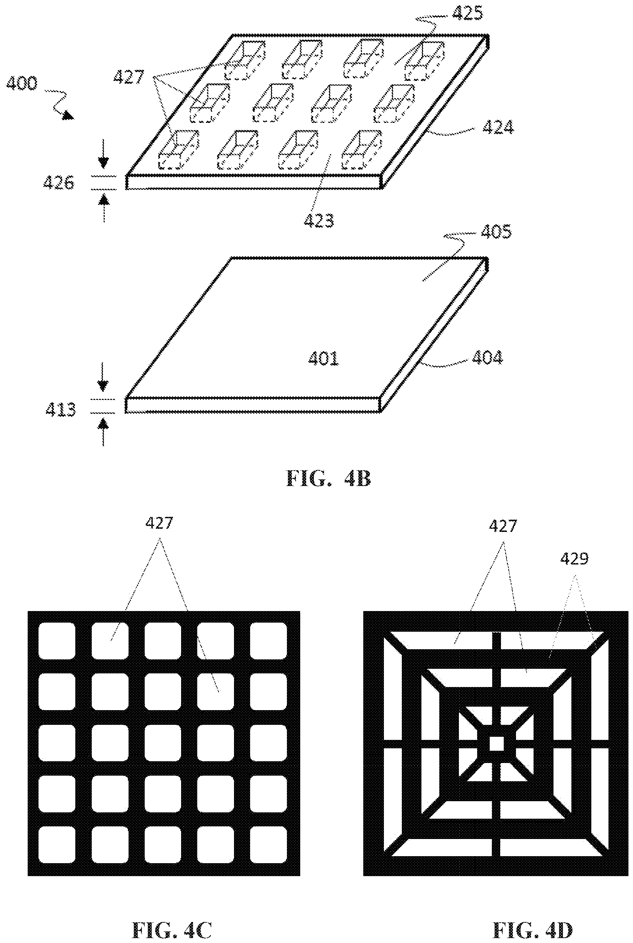

FIG. 4A illustrates a sectional view of a wound sealing film according to an example of the current invention. The wound sealing film 400, can include a first layer 401, and a combined layer 423. The first layer 401 can be any of the first layers or liquid barrier layers described herein. The combined layer 423 can be any of the combined layers described herein. FIG. 4B illustrates a perspective view of the wound sealing film 400. The first layer 401 can have a first surface 404 and second surface 405. The combined layer 423 can have a first surface 424 and second surface 425. The second surface 405 of first layer 401 can be in contact with the first surface 424 of the combined layer 423. The combined layer 423 can have a plurality of perforations 427. Perforations 427 of the combined layer 423 can be of difference shape and size. FIG. 4A and B illustrates a top view of the combined layer according to two examples of the current invention, the residual combined layer is shown in black and the perforations of the combined layer is shown in white.

The plurality of perforations 210 of the third layer 203 can contain perforations of any suitable shape and size. In some aspects, the plurality of perforations 210 of the third layer 203 can contain perforations of similar shape and/or size. In some aspects, the plurality of perforations 210 of the third layer 203 can contain perforations of different shape and/or size. Non limiting perforation shape includes, circular, oval, elliptical, square, rounded square, rectangular, rounded rectangular, pentagonal, rounded pentagonal, hexagonal, rounded hexagonal, heptagonal, rounded heptagonal, octagonal, rounded octagonal, star shaped, rounded star shaped, rod shaped or an irregular shaped. In some aspects, the plurality of perforations 210 of the third layer 203 can have an average cross-sectional length 212 of 2 mm to 50 mm or at least any one of, equal to any one of, or between any two of 2 mm, 4 mm, 5 mm, 6 mm, 8 mm, 10 mm, 12 mm, 14 mm, 16 mm, 18 mm, 20 mm, 22 mm, 24 mm, 26 mm, 28 mm, 30 mm, 32 mm, 34 mm, 35 mm, 36 mm, 38 mm, 40 mm, 42 mm, 44 mm, 46 mm, 48 mm and 50 mm. In some aspects, the third layer 203 forms ribs 250 defining the perforations, and the ribs have an average cross-section length parallel to the first surface and second surface of the second layer of 1 to 6 mm or at least any one of, equal to any one of, or between any two of 1 mm, 2 mm, 3 mm, 4 mm, 5 mm and 6 mm. In some aspects, the plurality of perforations 210 of the third layer 203 can form a repetitive pattern. In some aspects, the plurality of perforations 210 of the third layer 203 forms 25% to 98 %, or at least any one of, equal to any one of, or between any two of 25 %, 30 %, 35 %, 40 %, 45 %, 50 %, 55 %, 60 %, 65 %, 70 %, 75 %, 80 %, 85 %, 90 %, 95 %, and 98 % of the surface area of the third layer 203. The adjacent perforations of the plurality of the perforations 210 of the third layer 203 can have centers separated by 10 mm to 100 mm or at least any one of, equal to any one of, or between any two of 10 mm, 15 mm, 20 mm, 25 mm, 30 mm, 35 mm, 40 mm, 45 mm, 50 mm, 55 mm, 60 mm, 65 mm, 70 mm, 75 mm, 80 mm, 85 mm, 90 mm, 95 mm, and 100 mm.

The plurality of perforations 427 of the combined layer 423 can contain perforations of any suitable shape and size. In some aspects, the plurality of perforations 427 of the combined layer 423 can contain perforations of similar shape and/or size. In some aspects, the plurality of perforations 427 of the combined layer 423 can contain perforations of different shape and/or size. Non limiting perforation shape includes, circular, oval, elliptical, square, rounded square, rectangular, rounded rectangular, pentagonal, rounded pentagonal, hexagonal, rounded hexagonal, heptagonal, rounded heptagonal, octagonal, rounded octagonal, star shaped, rounded star shaped, rod shaped or an irregular shaped. In some aspects, the plurality of perforations 427 of the combined layer 423 can have an average cross-sectional length 428 of 2 mm to 50 mm or at least any one of, equal to any one of, or between any two of 2 mm, 4 mm, 5 mm, 6 mm, 8 mm, 10 mm, 12 mm, 14 mm, 16 mm, 18 mm, 20 mm, 22 mm, 24 mm, 26 mm, 28 mm, 30 mm, 32 mm, 34 mm, 35 mm, 36 mm, 38 mm, 40 mm, 42 mm, 44 mm, 46 mm, 48 mm and 50 mm. In some aspects, the combined layer 403 forms ribs 429 defining the perforations, and the ribs have an average cross-section length parallel to the first surface and second surface of the second layer of 1 to 6 mm or at least any one of, equal to any one of, or between any two of 1 mm, 2 mm, 3 mm, 4 mm, 5 mm and 6 mm. In some aspects, the plurality of perforations 427 of the combined layer 423 can form a repetitive pattern. In some aspects, the plurality of perforations 427 of the combined layer 423 forms 25% to 98 %, or at least any one of, equal to any one of, or between any two of 25 %, 30 %, 35 %, 40 %, 45 %, 50 %, 55 %, 60 %, 65 %, 70 %, 75 %, 80 %, 85 %, 90 %, 95 %, and 98 % of the surface area of the combined layer 423. The adjacent perforations of the plurality of the perforations 427 of the combined layer 423 can have centers separated by 10 mm to 100 mm or at least any one of, equal to any one of, or between any two of 10 mm, 15 mm, 20 mm, 25 mm, 30 mm, 35 mm, 40 mm, 45 mm, 50 mm, 55 mm, 60 mm, 65 mm, 70 mm, 75 mm, 80 mm, 85 mm, 90 mm, 95 mm, and 100 mm.

The first layer 101, 201, 301, 401 can contain a first polymer, such as the polymers of any of the first layers discussed herein and above. The first polymer can be polyurethane, polyethylene, cellulosics, polyamides, polyvinyl alcohol, polyvinyl pyrrolidone, acrylics, silicone elastomers, or copolymers of these. In some aspects, the first polymer can be polyurethane and the first layer 101, 201, 301, 401 can be formed from a polyurethane film. In some particular aspects, the first layer 101, 201, 301, 401 can be formed from a breathable cast matt polyurethane film sold by Expopack Advanced Coatings of Wrexham, United Kingdom under the name INSPIRE 2301 or INSPIRE 2327. In some aspects, width or thickness 113, 213, 313, 413 of the first layer 101, 201, 301, 401 can be 0.02 mm to 0.12 mm or at least any one of, equal to any one of, or between any two of 0.02 mm, 0.03 mm, 0.04 mm, 0.05 mm, 0.06 mm, 0.07 mm, 0.08 mm, 0.09 mm, 0.1 mm, 0.11 mm and 0.12 mm. In some aspects, the first layer 101, 201, 301, 401 can have a real weight 30 gsm to 100 gsm or at least any one of, equal to any one of, or between any two of 30 gsm, 40 gsm, 50 gsm, 60 gsm, 70 gsm, 80 gsm, 90 gsm, and 100 gsm.

The second layer 102, 202 can contain an adhesive. The adhesive can be any of the adhesives of the second layer disclosed herein and above. In some aspects, the adhesive can be a high tack acrylic adhesive and/or light switchable adhesive. In some aspects, width or thickness 114, 214 of the second layer 102, 202 can be 0.005, 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, or 2 mm. In some aspect, the light-switchable adhesive can be a light-switchable adhesive as described in US Provisional Application 62/858,089 by Locke et al..

In some aspects, the third layer 103, 203 can contain a polymeric gel. The polymer gel can be any of the polymeric gels of the third layer described herein and above. In some aspects, the polymeric gel can contain a polyurethane gel, a hydrogel, a silicone gel, a hydrocolloid, or a combination thereof. In some aspects, the hydrocolloid can include, a polymer, a first particle containing a water absorbing compound; and optionally a plasticizer. In some aspects, the first particle can be dispersed in a matrix of the polymer. In some aspects, the water absorbing compound can be carboxymethyl cellulose and/or a salt thereof. In some aspect, the polymer can contain a water sensitive polymer. In some particular aspects, the water sensitive polymer can contain pectin, carrageenan, gelatin, and/or alginate. In some aspect, the polymer can contain a water non-sensitive polymer. In some particular aspects, the water non-sensitive polymer can contain an isoprene polymer, a styrene-ethylene-butene-styrene (SEBS) block polymer, an isoprene isobutene copolymer, a styrene-isoprene-styrene copolymer, an ethylene vinyl acetate copolymer or any combination thereof. In some aspects, the plasticizer can be a paraffinic plasticizer, a naphthenic plasticizer, a petroleum jelly, an amorphous alpha olefin, a natural oil or any combination thereof. In some aspects, the paraffinic plasticizer can contain branched or non-branched saturated hydrocarbon chains, up to 50 carbon atoms long. In some aspects, the paraffinic plasticizer can contain branched or non-branched saturated hydrocarbon chains, up to 50 carbon atoms long, the saturated hydrocarbon chains can contain areas that can crystalize (wax). In some aspects, the paraffinic plasticizer can be aromatic-free paraffinic white mineral oils. In some aspects, the paraffinic plasticizer can be paraffinic process oils. In some aspects, the paraffinic plasticizer can be paraffinic process oils manufactured via the solvent extraction process. In some aspects, the paraffinic plasticizer can be refined hydrotreated paraffinic process oils. In some aspects, the paraffinic plasticizer can be refined hydrotreated paraffinic process oils and are essentially colorless and sulfur free. In some aspects, the naphthenic plasticizer can contain branched or non-branched saturated hydrocarbon chains, up to 50 carbon atoms long and contain cyclic or ring structure. In some aspects, the amorphous alpha olefin can be atactic polypropylene. In some aspects the natural oil can be linseed oil, soybean oil, tall oil or any combination thereof. In some aspects, the third layer 103, 203 can have a water absorption rate of 0.5 g/g to 5 g/g or at least any one of, equal to any one of, or between any two of 0.5 g/g, 1 g/g, 1.5 g/g, 2 g/g, 2.5 g/g, 3 g/g, 3.5 g/g, 4 g/g, 4.5 g/g, and 5 g/g. In some aspects, the third layer 103, 203 can have a dry adhesive strength of 5 N/25 cm to 15 N/25 cm or at least any one of, equal to any one of, or between any two of 5 N/25 cm, 7 N/25 cm, 9 N/25 cm, 11 N/25 cm, 13 N/25 cm, 15 N/25 cm, 17 N/25 cm, 19 N/25 cm, 21 N/25 cm, 23 N/25 cm, and 25 N/25 cm. In some aspects, moist adhesive strength of the third layer 103, 203 can be 15 % to 100 %, or at least any one of, equal to any one of, or between any two of 15 %, 20 %, 25%, 30 %, 35 %, 40 %, 45 %, 50 %, 55 %, 60 %, 65 %, 70 %, 75 %, 80 %, 85 %, 90 %, 95 %, or 100 % of the dry adhesive strength of the second layer 102, 202, 302. In some aspects, the third layer 103, 203 can have a density of 0.9 g/cc to 1.5 g/cc or at least any one of, equal to any one of, or between any two of 0.9 g/cc, 1 g/cc, 1.1 g/cc, 1.2 g/cc, 1.3 g/cc, 1.4 g/cc, and 1.5 g/cc. In some aspects, width or thickness 115, 215 of the third layer 103, 203 can be 0.1 mm to 1.2 mm or at least any one of, equal to any one of, or between any two of 0.1 mm, 0.2 mm, 0.3 mm, 0.4 mm, 0.5 mm, 0.6 mm, 0.7 mm, 0.8 mm, 0.9 mm, 1 mm, 1.1 mm and 1.2 mm. In some aspects, the third layer 103, 203 can have a hardness of 10 to 50 Shore 00, or at least any one of, equal to any one of, or between any two of 10 shore 00, 20 shore 00, 30 shore 00, 40 shore 00, and 50 shore 00, as measured by ASTM D2240 using a shore durometer. In some aspects, the third layer 103, 203 can be a transparent layer.

The combined layer 323, 423 can contain an adhesive and a polymeric gel. The adhesive and the polymeric gel can be any of the adhesives or polymeric gels disclosed in a combination layer herein or above. In some aspects, combined layer 323, 423 can be a homogenous mixture of the adhesive and the polymeric gel. In some aspects, the adhesive can be a high tack acrylic adhesive and/or light switchable adhesive. In some aspect, the light-switchable adhesive can be a light-switchable adhesive as described in US provisional application 62/858,089 by Locke et al.*.* In some aspects, the polymeric gel can contain a polyurethane gel, a hydrogel, a silicone gel, a hydrocolloid, or a combination thereof. In some aspects, the hydrocolloid can include, a polymer, a first particle containing a water absorbing compound; and optionally a plasticizer. In some aspects, the first particle can be dispersed in a matrix of the polymer. In some aspects, the water absorbing compound can be carboxymethyl cellulose and/or a salt thereof. In some aspect, the polymer can contain a water sensitive polymer. In some particular aspects, the water sensitive polymer can contain pectin, carrageenan, gelatin, and/or alginate. In some aspect, the polymer can contain a water non-sensitive polymer. In some particular aspects, the water non-sensitive polymer can contain an isoprene polymer, a styrene-ethylene-butene-styrene (SEBS) block polymer, an isoprene isobutene copolymer, a styrene-isoprene-styrene copolymer, an ethylene vinyl acetate copolymer or any combination thereof. In some aspects, the plasticizer can be a paraffinic plasticizer, a naphthenic plasticizer, a petroleum jelly, an amorphous alpha olefin, a natural oil or any combination thereof. In some aspects, the paraffinic plasticizer can contain branched or non-branched saturated hydrocarbon chains, up to 50 carbon atoms long. In some aspects, the paraffinic plasticizer can contain branched or non-branched saturated hydrocarbon chains, up to 50 carbon atoms long, the saturated hydrocarbon chains can contain areas that can crystalize (wax). In some aspects, the paraffinic plasticizer can be aromatic-free paraffinic white mineral oils. In some aspects, the paraffinic plasticizer can be paraffinic process oils. In some aspects, the paraffinic plasticizer can be paraffinic process oils manufactured via the solvent extraction process. In some aspects, the paraffinic plasticizer can be refined hydrotreated paraffinic process oils. In some aspects, the paraffinic plasticizer can be refined hydrotreated paraffinic process oils and are essentially colorless and sulfur free. In some aspects, the naphthenic plasticizer can contain branched or non-branched saturated hydrocarbon chains, up to 50 carbon atoms long and contain cyclic or ring structure. In some aspects, the amorphous alpha olefin can be atactic polypropylene. In some aspects the natural oil can be linseed oil, soybean oil, tall oil or any combination thereof.

In some aspects, the combined layer 323, 423 can have a water absorption rate of 0.05, 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12 g/g or any range thereof or rate therein, such as 0.1 g/g to 10 g/g. In some aspects, the combined layer 323, 423 can have a bond strength to steel of 0.5, 1, 2, 3, 4, 5, 6, 7, 8, 9, 10, 11, 12, 13, 14, 15, 16, 17, 18, 19, 20 N/25 mm or any range thereof or strength therein, such as 1 to 20, 5 to 15, 7 to 15, 7 to 14, 9 to 14, 8 to 14, 8 to 12, or 8 to 10 N/25 mm. In some aspects, the combined layer 323, 423 can have a dry adhesive strength of 0.5, 1, 2, 3, 4, 5, 6, 7, 8, 9, 10, 11, 12, 13, 14, 15, 16, 17, 18, 19, 20 N/25 mm or any range thereof or strength therein, such as 1 to 20, 5 to 15, 7 to 15, 7 to 14, 9 to 14, 8 to 14, 8 to 12, or 8 to 10 N/25 mm. In some aspects, moist adhesive strength of the combined layer 323, 423 can be 15 % to 100 %, or at least any one of, equal to any one of, or between any two of 15 %, 20 %, 25 %, 30 %, 35 %, 40 %, 45 %, 50 %, 55 %, 60 %, 65 %, 70 %, 75 %, 80 %, 85 %, 90 %, 95 %, or 100 % of the dry adhesive strength of the combined layer 323, 423. In some aspects, the combined layer 323, 423 can have a density of 0.8, 0.9, 1, 1.1, 1.2, 1.3, 1.4, 1.5, 1.6 g/cc or any range thereof or rate therein, such as 0.9 g/cc to 1.5 g/cc. In some aspects, width or thickness 326, 426 of the combined layer 323, 423 can be 0.1, 0.2, 0.3, 0.4, 0.5, 0.6, 0.7, 0.8, 0.9, 1.0, 1.1, 1.2, 1.4, 1.5, 1.6, 1.7, 1.8, 1.9, 2.0, 2.1, 2.2, 2.3, 2.4, 2.5, 2.6, 2.7, 2.8, 2.9, or 3 mm, or any range thereof or thickness therein thick, such as 0.1 mm to 2 mm thick. In some aspects, the combined layer 323, 423 can have a hardness of 5, 10, 15, 20, 25, 30, 35, 40, 45, 50, 55, 60 Shore 00 or any range thereof or hardness therein such as 10 to 50 Shore 00 as measured by ASTM D2240 using a shore durometer. In some aspects, the combined layer 323, 423 can have an areal weight of 50, 100, 200, 300, 400, 500, 600, 700, 800, 900, 1000, 1100, 1200, 1300 grams per square meter (gsm) or any range thereof or areal weight therein such as 100 to 1200 gsm. In some aspects, the combined layer 323, 423 can have a moisture vapor transfer rate (MVTR) of greater than 250 g/m²/24 hours. In some aspects, the combined layer can have a moisture vapor transfer rate (MVTR) of greater than 250 g/m²/24 hours, greater than 500 g/m²/24 hours, greater than 600 g/m²/24 hours, greater than 700 g/m²/24 hours, greater than 750 g/m²/24 hours, or at least 800 g/m²/24 hours. The moisture vapor transfer rate of the combined layer can be 250, 300, 350, 400, 450, 500, 550, 600, 650, 700, 750, 800, 850, 900, 950, 1000, 1100, 1200, 1300, 1400, 1500, 2000 g/m²/24 hours or any range thereof or weight therein. In some aspects, the combined layer 323, 423 can be a transparent layer.

In some aspects, the light switchable adhesive, including an adhesive polymeric gel, can comprise: one or more polymers; first photo initiators configured to cause the one or more polymers to cross-link responsive to receiving first light; and second photo initiators configured to cause the one or more polymers to cross-link responsive to receiving second light, the second photo initiators can be different from the first photo initiators. In some aspects, the light switchable adhesive has at least three phases, each phase corresponding to a particular peel strength, and wherein the light switchable adhesive is configured to transition between a first two phases of the three phases based on activation of the first photo initiators and to transition between a second two phases of the three phases based on activation of the second photo initiators. In some aspects, the light switchable adhesive has a second peel strength in the second phase that is greater than a first peel strength of the light switchable adhesive in the first phase, and wherein a third peel strength of the light switchable adhesive in the third phase is less than the second peel strength. In some aspects, the one or more polymers can be an acrylate polymer, urethane acrylate polymer, methyl acrylate polymer, silicone acrylate polymer, polyether, polyurethane, or any combination thereof. In some aspects, the one or more polymers can be an acrylate polymer or polyurethane polymer. In some aspects, the first photo initiators have a peak absorbance between 750 nanometers (nm) to 860 nm. In some aspects, the second photo initiators have a peak absorbance between 200 nanometers (nm) to 400 nm. In some particular aspects, the first photo initiators can include H-Nu-IR 780, H-Nu-IR 815, or both. In some particular aspects, the second photo initiators can include Irgacure 819. In some aspects, the light switchable adhesive can have a peel strength of less than 7 N/25 mm on stainless steel at an angle of 180 degrees in a first phase. In some aspects, the light switchable adhesive can have a peel strength of greater 8 N/25 mm on stainless steel at an angle of 180 degrees in a second phase. Additionally, or alternatively, the light switchable adhesive can have a peel strength of less than 7 N/25 mm on stainless steel at an angle of 180 degrees in a third phase. In some of the foregoing embodiments of the present compositions, the light switchable adhesive has a second level of cross-linking in the second phase that is greater than a first level of cross-linking in the first phase, and wherein the light switchable adhesive has a third level of cross-linking in the third phase that is greater than the second level of cross-linking.

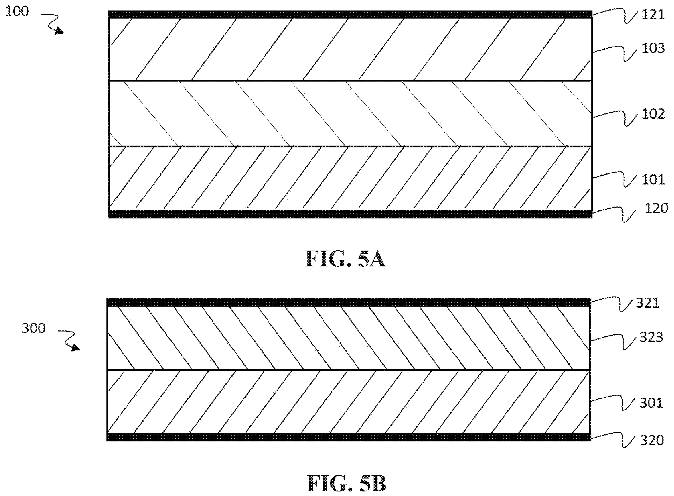

The wound sealing film 100, 200, 300, 400 can further contain a first cover layer and/or a second cover layer. The first cover layer can contact the first surface 104, 204, 304, 404 of the first layer 101, 201, 301, 401. The second cover layer can contact the second surface 109, 209 of the third layer 103, 203 or the second surface 325, 425 of the combined layer 323, 423. FIG. 5 illustrates wound sealing film 100 (A) with the first cover layer 120 and second cover layer 121, and wound sealing film 300 (B) with the first cover layer 320 and second cover layer 321.

In some aspects, moisture vapor transmission rate (MVTR) of the wound sealing film 100, 200, 300, 400 can be 300 g/m²/24 hours to 15000 g/m²/24 hours or at least any one of, equal to any one of, or between any two of 300 g/m²/24 hours, 500 g/m²/24 hours, 1000 g/m²/24 hours, 2000 g/m²/24 hours, 3000 g/m²/24 hours, 4000 g/m²/24 hours, 5000 g/m²/24 hours, 6000 g/m²/24 hours, 7000 g/m²/24 hours, 8000 g/m²/24 hours, 9000 g/m²/24 hours, 10000 g/m²/24 hours, 11000 g/m²/24 hours, 12000 g/m²/24 hours, 13000 g/m²/24 hours, 14000 g/m²/24 hours, and 15000 g/m²/24 hours. The wound sealing film 100, 200, 300, 400 can also function as a barrier to liquids and microorganisms.

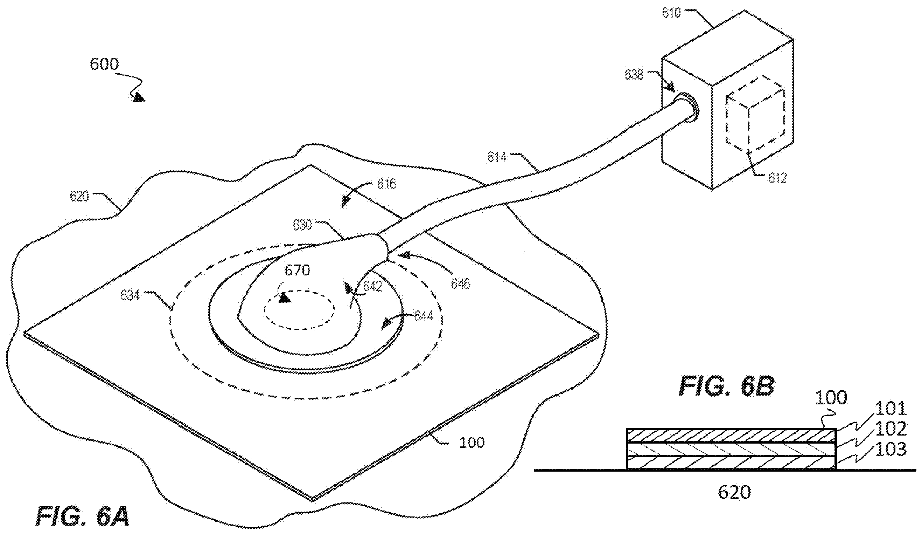

The wound sealing film 100, 200, 300, 400 may be configured to couple to a bandage, a wound closure device, a dressing, and/or a drape, to provide a seal to create an enclosed space (e.g., an interior volume) corresponding to a tissue site. For example, wound sealing film 100, 200, 300, 400 may be configured to provide a fluid seal (i.e., provide a portion of fluid seal) between two components and/or two environments, such as between a sealed therapeutic environment and a local ambient environment. To illustrate, when coupled to a tissue site, wound sealing film 100, 200, 300, 400 is configured to maintain a pressure differential at the tissue site and/or keep fluids from permeating through the wound sealing film 100, 200, 300, 400 as described further with reference to FIG. 6.

FIG. 6A shows a perspective view of an illustrative system 600 (e.g., a therapy system) for providing wound therapy. System 600 can include a wound sealing film 100 (shown), 200 (not shown), 300 (not shown), 400 (not shown), a therapy device 610, a canister 612, a tube 614, and a dressing 616. As an illustrative example, system 600 includes a wound sealing film 100, 200, 300, 400 as part of dressing 616.

System 600 is configured to provide therapy (e.g., oxygen therapy, positive-pressure therapy, negative-pressure therapy, or a combination thereof) at a tissue site 620 associated with a target area of a patient. For example, dressing 616 may be in fluid communication with tissue site 620 and may be in fluid communication with therapy device 610 via tube 614. In some implementations, system 600 may include one or more components commercially available through and/or from KCI USA, Inc. of San Antonio, Tex., U.S.A., and/or its subsidiary and related companies (collectively, "KCI").

Therapy device 610 (e.g., a treatment apparatus) is configured to provide therapy to tissue site 620 via tube 614 and dressing 616. For example, therapy device 610 may include a pressure source (e.g., a negative-pressure source, such as a pump, or a positive-pressure source, such as a pressurized oxygen container, an oxygen concentrator, or an oxygen collector) configured to be actuatable (and/or actuated) to apply pressure differential relative to ambient conditions to dressing 616. As illustrative, non-limiting examples, positive-pressure applied to a tissue site may typically range between 5 millimeters mercury (mm Hg) (667 pascals (Pa)) and 30 mm Hg (4.00 kilo (k) Pa). Common therapeutic ranges are between 10 mm Hg (1.33 kPa) and 25 mm Hg (3.33 kPa). As illustrative, non-limiting examples, reduced-pressure applied to a tissue site may typically ranges between -5 millimeters mercury (mm Hg) (-667 pascals (Pa)) and -500 mm Hg (-66.7 kilo (k) Pa). Common therapeutic ranges are between -75 mm Hg (-9.9 kPa) and -300 mm Hg (-39.9 kPa).

In some implementations, therapy device 610 may alternate between providing positive-pressure therapy and negative-pressure therapy to the dressing 616, may provide positive-pressure therapy to a first portion of the dressing 616 and negative-pressure therapy to a second portion of the dressing 616, may provide no positive or negative pressure, or a combination thereof. In some such implementations, the therapy device 610 can provide positive-pressure therapy and negative-pressure therapy to the dressing 616 at the same time (e.g., partially concurrently).

As illustrated in FIG. 6A, therapy device 610 includes canister 612 to receive fluid from tissue site 620 or to provide fluid to tissue site 620. Although canister 612 is illustrated as being internal to and/or integrated with therapy device 610, in other implementations, canister 612 can be external to therapy device 610 (not shown).

Therapy device 610 may also include one or more other components, such as a sensor, a processing unit (e.g., a processor), an alarm indicator, a memory, a database, software, a display device, a user interface, a regulator, and/or another component, that further facilitate positive-pressure therapy. Additionally, or alternatively, therapy device 610 may be configured to receive fluid, exudate, and or the like via dressing 616 and tube 614. Therapy device 610 may include one or connectors, such as a representative connector 638. Connector 638 is configured to be coupled to tube 614. Additionally, or alternatively, therapy device 610 may include one or more sensors, such a pressure sensor (e.g., a pressure transducer). The one or more sensors may be configured to enable therapy device 610 to monitor and/or sense a pressure associated with tube 614 and/or dressing 616.

Tube 614 includes one or more lumens (e.g., one or more through conduits), such as a single lumen conduit or multiple single-lumen conduits. Tube 614 (e.g., a least one of the one or more lumens) is configured to enable fluid communication between therapy device 610 and dressing 616. For example, fluid(s) and/or exudate can be communicated between therapy device 610 and dressing 616, and/or one or more pressure differentials (e.g., positive-pressure, negative pressure, or both) can be applied by therapy device 610 to dressing 616. As an illustrative, non-limiting illustration, tube 614 is configured to deliver at least pressurized oxygen from therapy device 610 to dressing 616 to establish positive-pressure. Communication of fluid(s) and application of a pressure differential can occur separately and/or concurrently.

In some implementations, tube 614 may include multiple lumens, such as a primary lumen (e.g., a positive-pressure/fluid lumen) for application of positive-pressure and/or communication of fluid, and one or more secondary lumens proximate to or around the primary lumen. The one or more secondary lumens (e.g., one or more ancillary/peripheral lumens) may be coupled to one or more sensors (of therapy device 610), coupled to one or more valves, as an illustrative, non-limiting example. Although tube 614 is described as a single tube, in other implementations, system 600 may include multiple tubes, such as multiple distinct tubes coupled to therapy device 610, dressing 616, or both.

As used herein, a "tube" broadly refers to a tube, pipe, hose, conduit, or other structure with one or more lumens adapted to convey fluid, exudate, and/or the like, between two ends. In some implementations, a tube may be an elongated, cylindrical structure with some flexibility; however, a tube is not limited to such a structure. Accordingly, tube may be understood to include a multiple geometries and rigidity. Tube 614 includes one or more lumens (e.g., one or more through conduits), such as a single lumen conduit or multiple single-lumen conduits. Tube 614 (e.g., a least one of the one or more lumens) is configured to enable fluid communication between therapy device 610 and dressing 616. For example, fluid(s) and/or exudate can be communicated between therapy device 610 and dressing 616, and/or one or more pressure differentials (e.g., positive-pressure, negative pressure, or both) can be applied by therapy device 610 to dressing 616. As an illustrative, non-limiting illustration, tube 614 is configured to deliver at least pressurized oxygen from therapy device 610 to dressing 616 to establish positive-pressure. Communication of fluid(s) and application of a pressure differential can occur separately and/or concurrently.

Dressing 616 includes a connector 630 (also referred to as a dressing connection pad or a pad), the wound sealing film 100 (or 200 or 300 or 400), and a manifold 634 (also referred to as a distribution manifold or an insert or fluid manifold). The wound sealing film 100 (or 200 or 300 or 400) may be coupled to connector 630. To illustrate, the wound sealing film 100 (or 200 or 300 or 400) may be coupled to connector 630 via an adhesive, a separate adhesive drape over at least a portion of connector 630 and at least a portion of the wound sealing film 100 (or 200 or 300 or 400), or a pressure sensitive adhesive, or a combination thereof, as illustrative, non-limiting examples.

The wound sealing film 100 (or 200 or 300 or 400) may be configured to couple dressing 616 at tissue site 620 and/or to provide a seal to create an enclosed space (e.g., an interior volume) corresponding to tissue site 620. For example, wound sealing film 100 (or 200 or 300 or 400) may be configured to provide a fluid seal between two components and/or two environments, such as between a sealed therapeutic environment and a local ambient environment. To illustrate, when coupled to tissue site 620, wound sealing film 100 (or 200 or 300 or 400) is configured to maintain a pressure differential (provided by a positive-pressure source or a negative-pressure source) at tissue site 620. The wound sealing film 100 (or 200 or 300 or 400) may be configured to be coupled to tissue site 620 via the adhesive of the third layer 103, 203 or combined layer 323, 423.

Referring to FIG. 6B, when in use, the wound sealing film 100 (or 200 or 300 or 400) is configured to be positioned on and/or near tissue site 620, and may be secured at the tissue site 620. When in use, the third layer 103, 203 or combined layer 323, 434 of the wound sealing film 100 (or 200 or 300 or 400) is configured to contact tissue site 620.

Referring to FIG. 6A, manifold 634 is configured to be positioned on and/or near tissue site 620, and may be secured at the tissue site 620, such as secured by the wound sealing film 100 (or 200 or 300 or 400). The manifold 634 can be position over a wound. The term "manifold" as used herein generally refers to a substance or structure that may be provided to assist in applying a pressure differential (e.g., positive-pressure differential or negative-pressure differential) to, delivering fluids to, or removing fluids and/or exudate from a tissue site and/or target tissue. The manifold typically includes a plurality of flow channels or pathways that distribute fluids provided to and removed from the tissue site. In an illustrative implementation, the flow channels or pathways are interconnected to improve distribution of fluids provided to or removed from the tissue site. Manifold 634 may be a biocompatible material that may be capable of being placed in contact with the tissue site and distributing positive and/or negative-pressure to the tissue site. Manifold 634 may include, without limitation, devices that have structural elements arranged to form flow channels, such as foam, cellular foam, open-cell foam, porous tissue collections, liquids, gels, and/or a foam that includes, or cures to include, flow channels, as illustrative, non-limiting examples. Additionally, or alternatively, the manifold may include polyethylene, a polyolefin, a polyether, polyurethane, a co-polyester, a copolymer thereof, a combination thereof, or a blend thereof.

In some implementations, manifold 634 is porous and may be made from foam, gauze, felted mat, or other material suited to a particular biological application. In a particular implementation, manifold 634 may be a porous foam and may include a plurality of interconnected cells or pores that act as flow channels. The foam (e.g., foam material) may be either hydrophobic or hydrophilic. As an illustrative, non-limiting example, the porous foam may be a polyurethane, open-cell, reticulated foam such as GranuFoam^{®} material manufactured by Kinetic Concepts, Incorporated of San Antonio, Tex.

In some implementations, manifold 634 is also used to distribute fluids such as medications, antibacterials, growth factors, and other solutions to the tissue site. Other layers may be included in or on manifold 634, such as absorptive materials, wicking materials, hydrophobic materials, and hydrophilic materials. In an implementation in which the manifold 634 includes a hydrophilic material, manifold 634 may be configured to wick fluid away from tissue site 620 and to distribute positive-pressure to tissue site 620. The wicking properties of manifold 634 may draw fluid away from the tissue site 620 by capillary flow or other wicking mechanisms. An illustrative, non-limiting example of a hydrophilic foam is a polyvinyl alcohol, open-cell foam such as V.A.C. WhiteFoam^{®} dressing available from Kinetic Concepts, Inc. of San Antonio, Tex. Other hydrophilic foams may include those made from polyether and/or foams that have been treated or coated to provide hydrophilicity.

In some implementations, manifold 634 is constructed from bioresorbable materials that do not have to be removed from tissue site 620 following use of the system 600. Suitable bioresorbable materials may include, without limitation, a polymeric blend of polylactic acid (PLA) and polyglycolic acid (PGA). The polymeric blend may also include without limitation polycarbonates, polyfumarates, and capralactones. Manifold 634 may further serve as a scaffold for new cell-growth, or a scaffold material may be used in conjunction with manifold 634 to promote cell-growth. A scaffold may be a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials. Although a manifold 634 is illustrated in FIG.6A, in other implementations, dressing 616 does not include manifold 634. In such implementations, wound sealing film 100 (or 200 or 300 or 400) of dressing 616 is coupled to connector 630.

Connector 630 can include a body 642 (e.g., a housing) and a base 644, and can be configured to be coupled to tube 614 via an interface 646 (e.g., a port). Base 644 can be configured to be coupled to dressing 616. For example, base 644 may be coupled, such as via an adhesive, and/or pressure to the wound sealing film 100 (or 200 or 300 or 400) and/or manifold 634. In some implementations, base 644 comprises a flange that is coupled to an end of body 642 and/or is integrally formed with body 642. Connector 630, such as body 642, base 644, interface 646, or a combination thereof, may be made of rigid material and/or a semi-rigid material. In a non-limiting example, connector 630 may be made from a plasticized polyvinyl chloride (PVC), polyurethane, cyclic olefin copolymer elastomer, thermoplastic elastomer, poly acrylic, silicone polymer, or polyether block amide copolymer. In some implementations, connector 630 is formed of a semi-rigid material that is configured to expand when under a force, such as positive-pressure greater than or equal to a particular amount of pressure. Additionally or alternatively, connector 630 may be formed of a semi-rigid material that is configured to collapse when under a force, such as reduced-pressure less than or equal to a threshold pressure.

Body 642 includes one or more channels or one or more conduits that extend from and/or are coupled to interface 646. To illustrate, body 642 may include a primary channel configured to be coupled in fluid communication with a primary lumen of tube 614. The primary channel may be coupled to a cavity (e.g., a tissue cavity partially defined by body 642) having an aperture open towards manifold 634 (and/or towards tissue site 620). For example, the primary channel may include a first opening associated with interface 646 and a second opening (distinct from the aperture of the cavity) associated with the cavity. Thus, the primary channel may define a through channel of body 642 to enable fluid communication between interface 646 and tissue site 620.

Body 642 includes a channel (e.g., a through channel) having a first aperture open opposite dressing 616 and a second aperture open towards dressing 616. For example, the first aperture is located on an outer surface side (e.g., an ambient environment surface) of connector 630 and the second aperture is located on an inner surface side (e.g., a tissue facing side) of connector 630. Illustrative, non-limiting examples of commercially available connectors include a "V.A.C. T.R.A.C.^{®} Pad," or "Sensa T.R.A.C.^{®} Pad" available from Kinetic Concepts, Inc. (KCI) of San Antonio, Tex.

In some implementations. dressing 616 further includes a bandage and/or a wound closure device (not shown). For example, a bandage may be placed over a wound to protect the wound and a wound closure device may be placed proximate to a wound to provide a force to maintain tissue in fixed position to promote wound closure.

During operation of system 600, dressing 616 is coupled to tissue site 620 over a wound. Additionally, dressing 616 is coupled to device 610 via tube 614. In some implementations, prior to coupling the dressing 616 to the tissue site 620, a manifold 634 is coupled to tissue site 620 proximate to or over the wound. The dressing 616 with the wound sealing film 100 (or 200 or 300 or 400) is then coupled over the manifold 634 to seal the wound. In some aspects, an aperture 670 can be made in the wound sealing film 100 (or 200 or 300 or 400) over the manifold, where the aperture 670 that extends through wound sealing film 100 (or 200 or 300 or 400) enables a fluid communication between therapy device 610 and the wound in the tissue site through the manifold 634. In some aspects, the wound sealing film 100 (or 200 or 300 or 400) can have a preformed aperture 670, wherein the aperture is placed over a manifold 634 over a wound. In some aspects, a wound contact layer (not shown) is placed between the wound surface a the manifold.

A pressure differential, such as positive-pressure, can be generated and/or applied to dressing 616 (e.g., the interior volume of dressing 616) by a pressure source associated with device 610. When positive-pressure is generated and/or applied to dressing 616, fluid or medication from device 610, such as from canister 612, may be transported to the wound in the tissue site 620 through the dressing 616. Furthermore, in some implementations, reduced-pressure can be applied to the wound in the tissue site 620 through the dressing 616 by a reduced-pressure source associated with device 610. When reduced-pressure is applied, fluid, exudate, or other material from the wound in the tissue site 620 may be transported to canister 612 of device 610.

## Claims

1. A wound sealing film comprising:
a first layer having a first surface and a second surface, said first layer comprising a first polymer composition;
a second layer having a first surface and a second surface, said second layer comprising an adhesive; and
a third layer having a first surface and a second surface, said third layer comprising a polymeric gel,
wherein the second layer is positioned between the first layer and the third layer,
the third layer comprising a plurality of perforations defined by ribs having an average cross-section length parallel to the first surface and the second surface of the third layer of between 1 to 6 mm.

2. The wound sealing film of claim 1, wherein the plurality of perforations of the third layer have an average cross-sectional length of about 2 mm to about 50 mm.

3. The wound sealing film of any one of claims 1 or 2, wherein the plurality of perforations of the third layer comprises adjacent perforations having centers separated by 5 mm to 100 mm.

4. The wound sealing film of any one of claims 1 to 3, wherein the plurality of perforations of the third layer forms 25% to 98% of the surface area of the third layer.

5. The wound sealing film of any one of claims 1 to 4, wherein the adhesive is a high tack acrylic adhesive, a color changing adhesive, and/or light-switchable adhesive.

6. The wound sealing film of any one of claims 1 to 5, wherein the polymeric gel comprises a polyurethane gel, polyurethane adhesive, a hydrogel, a hydrocolloid or any combination thereof.

7. The wound sealing film of claim 6, wherein the hydrocolloid comprises:
a polymer;
a first particle comprising a water absorbing compound; and
optionally a plasticizer.

8. The wound sealing film of claim 7, wherein the first particle is dispersed in a matrix of the polymer.

9. The wound sealing film of any of one claims 7 or 8, wherein the water absorbing compound is carboxymethyl cellulose and/or a salt thereof.

10. The wound sealing film of any of one claims 7 to 9, wherein the polymer comprises a water sensitive polymer.

11. The wound sealing film of claim 10, wherein the water sensitive polymer comprises starch, acrylic polymers, acrylic-sulphonic acid polymers such as 2-acrylamido-2-methylpropane sulfonic acid polymer, pectin, carrageenan, gelatin, and/or alginate.

12. The wound sealing film of any of one claims 7 to 11, wherein the polymer comprises a water non-sensitive polymer.

13. The wound sealing film of claim 12, wherein the water non-sensitive polymer comprises an isoprene polymer, polyisobutylene, ethylene-propylene copolymer, ethylene propylene diene monomer (EPDM) copolymer, a styrene-ethylene-butene-styrene (SEBS) block polymer, an isoprene isobutene copolymer, a styrene-isoprene-styrene copolymer such as styrene-isoprene-styrene terpolymers, or an ethylene vinyl acetate copolymer.

14. The wound sealing film of any of one claims 7 to 13, wherein the plasticizer is a paraffinic plasticizer, a naphthenic plasticizer, a petroleum jelly, an amorphous alpha olefin, a natural oil or any combination thereof.

15. The wound sealing film of any one of claims 1 to 14, wherein the third layer is a color changing adhesive layer and/or a light switchable adhesive layer.

## Patentansprüche

1. Ein Wundverschlussfilm, aufweisend:
eine erste Schicht, die eine erste Oberfläche und eine zweite Oberfläche vorweist, die erste Schicht aufweisend eine erste Polymerzusammensetzung;
eine zweite Schicht, die eine erste Oberfläche und eine zweite Oberfläche vorweist, die zweite Schicht aufweisend einen Klebstoff; und
eine dritte Schicht, die eine erste Oberfläche und eine zweite Oberfläche vorweist, die dritte Schicht aufweisend ein Polymergel,
wobei die zweite Schicht zwischen der ersten Schicht und der dritten Schicht angeordnet ist,
die dritte Schicht aufweisend eine Mehrzahl von Perforationen, die durch Rippen definiert sind, die parallel zu der ersten Oberfläche und der zweiten Oberfläche der dritten Schicht eine durchschnittliche Querschnittslänge zwischen 1 bis 6 mm vorweisen.

2. Der Wundverschlussfilm nach Anspruch 1, wobei die Mehrzahl von Perforationen der dritten Schicht eine durchschnittliche Querschnittslänge von etwa 2 mm bis etwa 50 mm vorweist.

3. Der Wundverschlussfilm nach einem der Ansprüche 1 oder 2, wobei die Mehrzahl von Perforationen der dritten Schicht angrenzende Perforationen aufweist, die Mittelpunkte vorweisen, die um 5 mm bis 100 mm getrennt sind.

4. Der Wundverschlussfilm nach einem der Ansprüche 1 bis 3, wobei die Mehrzahl von Perforationen der dritten Schicht 25 % bis 98 % des Oberflächenbereichs der dritten Schicht ausbildet.

5. Der Wundverschlussfilm nach einem der Ansprüche 1 bis 4, wobei der Klebstoff ein Acrylatklebstoff mit hoher Klebrigkeit, ein farbwechselnder Klebstoff und/oder ein durch Licht veränderbarer Klebstoff ist.

6. Der Wundverschlussfilm nach einem der Ansprüche 1 bis 5, wobei das Polymergel ein Polyurethangel, einen Polyurethanklebstoff, ein Hydrogel, ein Hydrokolloid oder eine beliebige Kombination davon aufweist.

7. Der Wundverschlussfilm nach Anspruch 6, wobei das Hydrokolloid aufweist:
ein Polymer;
ein erstes Partikel, aufweisend eine wasserabsorbierende Verbindung; und
optional einen Weichmacher.

8. Der Wundverschlussfilm nach Anspruch 7, wobei das erste Partikel in einer Matrix des Polymers dispergiert ist.

9. Der Wundverschlussfilm nach einem der Ansprüche 7 oder 8, wobei die wasserabsorbierende Verbindung Carboxymethylcellulose und/oder ein Salz davon ist.

10. Der Wundverschlussfilm nach einem der Ansprüche 7 bis 9, wobei das Polymer ein wasserempfindliches Polymer aufweist.

11. Der Wundverschlussfilm nach Anspruch 10, wobei das wasserempfindliche Polymer Stärke, Acrylpolymere, Acrylsulfonsäurepolymere wie etwa 2-Acrylamido-2-methylpropansulfonsäurepolymer, Pektin, Carrageenan, Gelatine und/oder Alginat aufweist.

12. Der Wundverschlussfilm nach einem der Ansprüche 7 bis 11, wobei das Polymer ein wasserunempfindliches Polymer aufweist.

13. Der Wundverschlussfilm nach Anspruch 12, wobei das wasserunempfindliche Polymer ein Isoprenpolymer, Polyisobutylen, ein Ethylen-Propylen-Copolymer, Ethylen-Propylen-Dien-Monomer-Copolymer (EPDM-Copolymer), ein Styrol-Ethylen-Buten-Styrol-Blockpolymer (SEBS-Blockpolymer), ein Isopren-Isobuten-Copolymer, ein Styrol-Isopren-Styrol-Copolymer wie etwa Styrol-Isopren-Styrol-Terpolymere oder ein Ethylen-Vinylacetat-Copolymer aufweist.

14. Der Wundverschlussfilm nach einem der Ansprüche 7 bis 13, wobei der Weichmacher ein paraffinischer Weichmacher, ein naphthenischer Weichmacher, eine Vaseline, ein amorphes Alpha-Olefin, ein natürliches Öl oder eine beliebige Kombination davon ist.

15. Der Wundverschlussfilm nach einem der Ansprüche 1 bis 14, wobei die dritte Schicht eine farbwechselnde Klebstoffschicht und/oder eine durch Licht veränderbare Klebstoffschicht ist.

## Revendications

1. Film d'étanchéité de plaie comprenant :
une première couche ayant une première surface et une seconde surface, ladite première couche comprenant une première composition de polymère ;
une deuxième couche ayant une première surface et une seconde surface, ladite deuxième couche comprenant un adhésif ; et
une troisième couche ayant une première surface et une seconde surface, ladite troisième couche comprenant un gel polymère,
dans lequel la deuxième couche est positionnée entre la première couche et la troisième couche,
la troisième couche comprenant une pluralité de perforations définies par des nervures ayant une longueur en coupe transversale moyenne parallèle à la première surface et à la seconde surface de la troisième couche, comprise de 1 à 6 mm.

2. Film d'étanchéité de plaie selon la revendication 1, dans lequel la pluralité de perforations de la troisième couche ont une longueur en coupe transversale moyenne d'environ 2 mm à environ 50 mm.

3. Film d'étanchéité de plaie selon l'une quelconque des revendications 1 ou 2, dans lequel la pluralité de perforations de la troisième couche comprend des perforations adjacentes ayant des centres séparés par 5 mm à 100 mm.

4. Film d'étanchéité de plaie selon l'une quelconque des revendications 1 à 3, dans lequel la pluralité de perforations de la troisième couche forme 25 % à 98 % de la superficie de la troisième couche.

5. Film d'étanchéité de plaie selon l'une quelconque des revendications 1 à 4, dans lequel l'adhésif est un adhésif acrylique à fort pouvoir collant, un adhésif à changement de couleur, et/ou un adhésif commutable à la lumière.

6. Film d'étanchéité de plaie selon l'une quelconque des revendications 1 à 5, dans lequel le gel polymère comprend un gel de polyuréthane, un adhésif de polyuréthane, un hydrogel, un hydrocolloïde ou n'importe quelle combinaison de ceux-ci.

7. Film d'étanchéité de plaie selon la revendication 6, dans lequel l'hydrocolloïde comprend :
un polymère ;
une première particule comprenant un composé absorbant l'eau ; et
facultativement un plastifiant.

8. Film d'étanchéité de plaie selon la revendication 7, dans lequel la première particule est dispersée dans une matrice du polymère.

9. Film d'étanchéité de plaie selon l'une quelconque des revendications 7 ou 8, dans lequel le composé absorbant l'eau est de la carboxyméthylcellulose et/ou un sel de celle-ci.

10. Film d'étanchéité de plaie selon l'une quelconque des revendications 7 à 9, dans lequel le polymère comprend un polymère sensible à l'eau.

11. Film d'étanchéité de plaie selon la revendication 10, dans lequel le polymère sensible à l'eau comprend amidon, polymères acryliques, polymères d'acide acrylique-sulfonique tels que polymère d'acide 2-acrylamido-2-méthylpropane sulfonique, pectine, carraghénane, gélatine et/ou alginate.

12. Film d'étanchéité de plaie selon l'une quelconque des revendications 7 à 11, dans lequel le polymère comprend un polymère non sensible à l'eau.

13. Film d'étanchéité de plaie selon la revendication 12, dans lequel le polymère non sensible à l'eau comprend polymère d'isoprène, polyisobutylène, copolymère d'éthylène-propylène, copolymère d'éthylène-propylène-monomère diénique (EPDM), polymère séquencé de styrène-éthylène-butène-styrène (SEBS), copolymère isoprène-isobutène, copolymère de styrène-isoprène-styrène tel que terpolymères de styrène-isoprène-styrène, ou copolymère d'éthylèneacétate de vinyle.

14. Film d'étanchéité de plaie selon l'une quelconque des revendications 7 à 13, dans lequel le plastifiant est un plastifiant paraffinique, un plastifiant naphténique, une vaseline, une alpha oléfine amorphe, une huile naturelle ou n'importe quelle combinaison de ceux-ci.

15. Film d'étanchéité de plaie selon l'une quelconque des revendications 1 à 14, dans lequel la troisième couche est une couche d'adhésif à changement de couleur et/ou une couche d'adhésif commutable à la lumière.
